# EUROPÄISCHE PATENTANMELDUNG

(11) **EP 2 277 870 A1**
(43) Veröffentlichungstag der Anmeldung: **26.01.2011**
(21) Anmeldenummer: 09163556.5
(22) Anmeldetag: 24.06.2009
(51) Int. Cl.: C07D 263/56, C07D 263/60, C07C 205/60, C07C 227/04, C07C 229/60, A01N 43/76, A01P 3/00

(54) **Substituierte Benzoxa(thia)zole**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft neue substituierte Benzoxa(thia)zole, Verfahren zum Herstellen dieser Verbindungen, Mittel enthaltend diese Verbindungen, sowie deren Verwendung als biologisch aktive Verbindungen, insbesondere zur Bekämpfung von schädlichen Mikroorganismen im Pflanzenschutz und im Materialschutz.

## Beschreibung

Die vorliegende Erfindung betrifft neue substituierte Benzoxa(thia)zole, Verfahren zum Herstellen dieser Verbindungen, Mittel enthaltend diese Verbindungen, sowie deren Verwendung als biologisch aktive Verbindungen, insbesondere zur Bekämpfung von schädlichen Mikroorganismen im Pflanzenschutz und im Materialschutz.

Es ist bereits bekannt, dass bestimmte Benzoxazole oder Benzthiazole im Pflanzenschutz als Fungizide eingesetzt werden können (vgl. JP 2005-145840 und WO 1997/08170). Außerdem sind bestimmte Benzoxazole als 5-HT₃-Inhibitoren bekannt (vgl. WO 2008/019363).

Da sich die ökologischen und ökonomischen Anforderungen an moderne Wirkstoffe, z.B. Fungizide, laufend erhöhen, beispielsweise bezüglich Wirkspektrum, Toxizität, Selektivität, Aufwandmenge, Rückstandsbildung und günstige Herstellbarkeit, und außerdem z.B. Probleme mit Resistenzen auftreten können, besteht die ständige Notwendigkeit, neue fungizide Mittel zu entwickeln, die zumindest in Teilbereichen Vorteile gegenüber den bekannten aufweisen.

Es wurden nun neue Benzoxa(thia)zole der Formel (I) 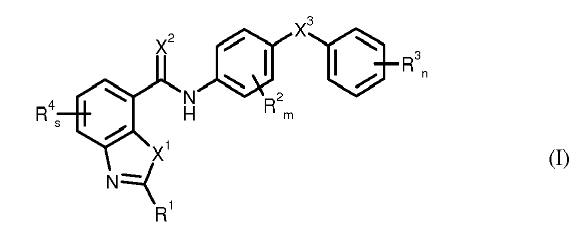 gefunden, in welcher
- X¹: für Sauerstoff oder Schwefel steht,
- X²: für Sauerstoff oder Schwefel steht,
- X³: für Sauerstoff oder Schwefel steht,
- R¹: für Wasserstoff, Halogen, C₁-C₄-Alkyl, Hydroxy, Sulfanyl (SH), C₁-C₈-Alkoxy, (C₁-C₈-Alkoxy)carbonyl, Amino, C₁-C₈-Alkylamino, Di-(C₁-C₈-Alkyl)amino, C₁-C₈-Alkylthio, C₁-C₈-Alkylsulfinyl oder C₁-C₈-Alkylsulfonyl steht,
- R²: für Halogen, Hydroxy, Cyano, Nitro, C₁-C₈-Alkyl, C₁-C₈-Alkoxy, C₁-C₈-Alkylthio, C₁-C₈-Alkyl-sulfinyl, C₁-C₈-Alkylsulfonyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl steht,
- m: für 0, 1, 2, 3 oder 4 steht, wobei R² gleich oder verschieden sein kann, wenn m für 2, 3 oder 4 steht,
- R³: für Halogen, Hydroxy, Cyano, Nitro, C₁-C₈-Alkyl, C₁-C₈-Alkoxy, C₁-C₈-Alkylthio, C₁-C₈-Alkylsulfinyl, C₁-C₈-Alkylsulfonyl, C₁-C₄-Alkoxy-C₁-C₄-alkyl, (C₁-C₄-Alkyl)thio(C₁-C-₄-alkyl), (C₁-C₄-Alkyl)sulfinyl(C₁-C-₄-alkyl), (C₁-C₄-Alkyl)sulfonyl(C₁-C₄-alkyl), C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl steht,
- n: für 0, 1, 2, 3, 4 oder 5 steht, wobei R³ gleich oder verschieden sein kann, wenn n für 2, 3, 4 oder 5 steht,
- R⁴: für Halogen, Hydroxy, Cyano, Nitro, C₁-C₈-Alkyl, C₁-C₈-Alkoxy, C₁-C₈-Alkylthio, C₁-C-₈-Alkylsulfinyl, C₁-C-₈-Alkylsulfonyl, C₁-C₄-Alkoxy-C₁-C₄-alkyl, (C₁-C₄-Alkyl)thio(C₁-C-₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Phenyl (welches wiederum gegebenenfalls einfach oder mehrfach, gleich oder verschieden durch Halogen, Cyano, Nitro, C₁-C₆-Alkyl, C₁-C₆-Alkoxy, C₁-C₆-Alkylthio, C₁-C₆-Halogenalkyl, C₁-C₆-Halogenalkylthio substituiert sein kann), Heteroaryl, Heterocyclyl steht,
- s: für 1 oder 2 steht, wobei zwei benachbarte Reste R² für C₄-Alkenylen stehen, wenn s für 2 steht,
sowie deren agrochemisch wirksamen Salze.

Je nach Art der oben definierten Substituenten weisen die Verbindungen der Formel (I) saure oder basische Eigenschaften auf und können mit anorganischen oder organischen Säuren oder mit anorganischen oder organischen Basen oder mit Metallionen Salze, gegebenenfalls auch innere Salze oder Addukte bilden. Als Metallionen kommen insbesondere die Ionen der Elemente der zweiten Hauptgruppe, insbesondere Calcium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkylresten, Mono-, Di- und Trialkanolamine von C₁-C₄-Alkanolen, Cholin sowie Chlorcholin.

Tragen die Verbindungen der Formel (I) Amino, Alkylamino oder andere, basische Eigenschaften induzierende Gruppen, so können diese Verbindungen mit Säuren zu Salzen umgesetzt werden. Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Die so erhältlichen Salze weisen ebenfalls fungizide Eigenschaften auf.

Die erfindungsgemäß verwendbaren Benzoxa(thia)zole sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- X¹: steht bevorzugt für Sauerstoff.
- X¹: steht außerdem bevorzugt für Schwefel.
- X¹: steht besonders bevorzugt für Sauerstoff.
- X²: steht bevorzugt für Sauerstoff.
- X²: steht außerdem bevorzugt für Schwefel.
- X²: steht besonders bevorzugt für Sauerstoff.
- X³: steht bevorzugt für Sauerstoff.
- X³: steht außerdem bevorzugt für Schwefel.
- X³: steht besonders bevorzugt für Sauerstoff.
- R¹: steht bevorzugt für Wasserstoff, Chlor, Sulfanyl (SH), Amino, C₁-C₆-Alkylamino oder C₁-C₆-Alkylthio.
- R¹: steht besonders bevorzugt für Wasserstoff oder Sulfanyl.
- R¹: steht ganz besonders bevorzugt für Wasserstoff.
- R²: steht bevorzugt für Fluor, Chlor, Brom, Cyano, Nitro, Methyl, Ethyl, n-Propyl, i-Propyl, n-, i-, s- oder t- Butyl, Methoxy, Ethoxy, Trifluormethyl, Difluormethyl, Trifluormethoxy oder Difluormethoxy.
- R²: steht besonders bevorzugt für Fluor, Chlor, Brom, Cyano, Nitro, Methoxy oder Trifluormethyl.
- m: steht bevorzugt für 0, 1, 2 oder 3, wobei R² gleich oder verschieden sein kann, wenn m für 2 oder 3 steht.
- m: steht besonders bevorzugt für 0, 1 oder 2, wobei R² gleich oder verschieden sein kann, wenn m für 2 steht.
- m: steht ganz besonders bevorzugt für 0.
- m: steht außerdem ganz besonders bevorzugt für 1.
- R³: steht bevorzugt für Fluor, Chlor, Brom, Iod, Cyano, Nitro, Methyl, Ethyl, n-Propyl, i-Propyl, n-, i-, s- oder t- Butyl, Methoxy, Ethoxy, Trifluormethyl, Difluormethyl, Trifluormethoxy oder Difluormethoxy.
- R³: steht besonders bevorzugt für Fluor, Chlor, Brom, Cyano, Nitro, Methyl, Methoxy oder Trifluormethyl.
- n: steht bevorzugt für 0, 1, 2 oder 3, wobei R³ gleich oder verschieden sein kann, wenn n für 2 oder 3 steht.
- n: steht besonders bevorzugt für 0, 1 oder 2, wobei R³ gleich oder verschieden sein kann, wenn n für 2 steht.
- n: steht ganz besonders bevorzugt für 0.
- n: steht außerdem ganz besonders bevorzugt für 1.
- R⁴: steht bevorzugt für Fluor, Chlor, Brom, Iod, Cyano, Nitro, Methyl, Ethyl, n-Propyl, i-Propyl, n-, i-, s- oder t- Butyl, Methoxy, Ethoxy, n-Propoxy, i-Propoxy, n-, i-, s- oder t- Butoxy, Methylthio, Ethylthio, n-Propylthio, i-Propylthio, n-, i-, s- oder t- Butylthio, Dimethylamino, Trifluormethyl, Trichlormethoxy, Trifluormethoxy oder Heteroaryl (welches bevorzugt ausgewählt ist aus 1-Inüdazolyl, 3-Thiazolyl, 3-Oxazolyl, 2-Pyridyl, 3-Pyridyl, 4-Pyridyl).
- R⁴: steht besonders bevorzugt für Fluor, Chlor, Brom, Iod, Cyano, Nitro, Methyl, Methoxy oder Trifluormethoxy.
- s: steht bevorzugt für 1.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel (I), in welcher alle Reste jeweils die oben genannten bevorzugten Bedeutungen haben.
Besonders bevorzugt sind solche Verbindungen der Formel (I), in welcher alle Reste jeweils die oben genannten besonders bevorzugten Bedeutungen haben.
Bevorzugt sind Verbindungen der Formel (I), in welcher X¹ für Sauerstoff steht.
Weiterhin bevorzugt sind Verbindungen der Formel (I), in welcher X² für Sauerstoff steht.
Weiterhin bevorzugt sind Verbindungen der Formel (I), in welcher X³ für Sauerstoff steht.
Weiterhin bevorzugt sind Verbindungen der Formel (I), in welcher X¹ und X² jeweils für Sauerstoff stehen.
Weiterhin bevorzugt sind Verbindungen der Formel (I), in welcher X¹ und X³ jeweils für Sauerstoff stehen.
Weiterhin bevorzugt sind Verbindungen der Formel (I), in welcher X³ und X² jeweils für Sauerstoff stehen.
Weiterhin bevorzugt sind Verbindungen der Formel (I), in welcher X¹, X² und X³ jeweils für Sauerstoff stehen.
Weiterhin bevorzugt sind Verbindungen der Formel (I), in welcher R¹ für Wasserstoff steht.
Weiterhin bevorzugt sind Verbindungen der Formel (I), in welcher X¹, X² und X³ jeweils für Sauerstoff und R¹ für Wasserstoff stehen.
Weiterhin bevorzugt sind Verbindungen der Formel (I), in welcher R² für Wasserstoff steht.
Weiterhin bevorzugt sind Verbindungen der Formel (I), in welcher R³ für Fluor, Chlor, Brom, Iod, Methyl, Ethyl, n-Propyl, i-Propyl, n-, i-, s- oder t- Butyl, Nitro, Cyano, Trifluormethyl, Difluormethyl, Methoxy, Ethoxy, Trifluormethoxy oder Difluormethoxy steht.
Weiterhin bevorzugt sind Verbindungen der Formel (I), in welcher R³ für Chlor, Fluor, Brom, Methyl, Nitro, Cyano, Methoxy, Trifluormethyl steht.
Weiterhin bevorzugt sind Verbindungen der Formel (I), in welcher m für 0 steht.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod;
**Alkyl:** gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; Heptyl, Octyl.
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl.
**Alkenyl:** ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.
**Alkinyl:** geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist.
**Heterocyclyl/Hetaryl:** unsubstituierter oder substituierter, ungesättigter oder ganz oder teilweise gesättigter heterocyclischer 5- bis 7-gliedrigen Ring, oder ungesättigter oder ganz oder teilweise gesättigter heterocyclischer 3- bis 8-gliedriger Ring, enthaltend bis zu 4 Stickstoffatome oder alternativ 1 Stickstoffatom und bis zu 2 weitere Heteroatome, ausgewählt aus N, O und S: z.B. Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 1-Pyrrolyl, 1-Pyrazolyl, 1,2,4-Triazol-1-yl, 1-Imidazolyl, 1,2,3-Triazol-1-yl, 1,3,4-Triazol-1-yl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.
**Alkoxy:** gesättigte, geradkettige oder verzweigte Alkoxyreste mit 1 bis 4 Kohlenstoffatomen, wie beispielsweise (aber nicht beschränkt auf) C₁-C₄-Alkoxy wie Methoxy, Ethoxy, Propoxy, 1-Methylethoxy, Butoxy, 1-Methyl-propoxy, 2-Methylpropoxy, 1,1-Dimethylethoxy;
**Haloalkoxy:** geradkettige oder verzweigte Alkoxygruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, wie beispielsweise (aber nicht beschränkt auf) C₁-C₂-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Alkylthio:** gesättigte, geradkettige oder verzweigte Alkylthioreste mit 1 bis 6 Kohlenstoffatomen, wie beispielsweise (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Haloenalkylthio:** geradkettige oder verzweigte Alkylthiogruppen mit 1 bis 6 Kohlenstoffatomen (wie vorstehend genannt), wobei in diesen Gruppen teilweise oder vollständig die Wasserstoffatome durch Halogenatome wie vorstehend genannt ersetzt sein können, wie beispielsweise (aber nicht beschränkt auf) C₁-C₂-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Cycloalkyl:** mono-, bi- oder tricyclische, gesättigte Kohlenwasserstoffgruppen mit 3 bis 12 Kohlenstoffringgliedern, wie z.B. (aber nicht beschränkt auf) Cyclopropyl, Cyclobutyl, Cyclopentyl und Cyclohexyl, Bicyclo[1,0,1]butan, Decalinyl Norbomyl.

### Erläuterung der Verfahren und Zwischenprodukte

Die substituierten Benzoxa(thia)zole der Formel (I) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen. 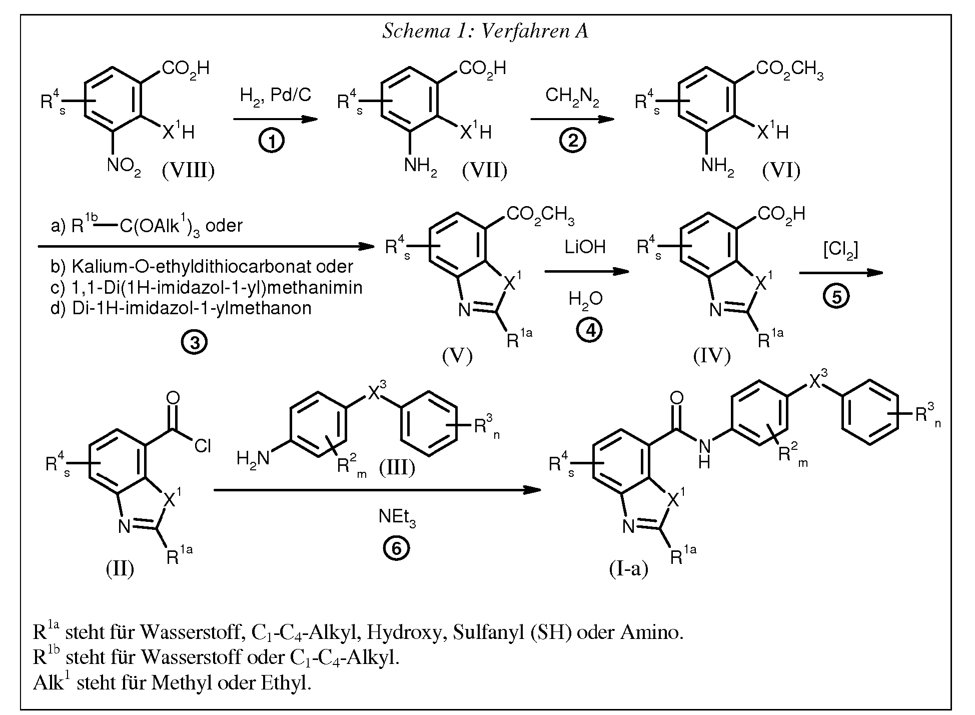 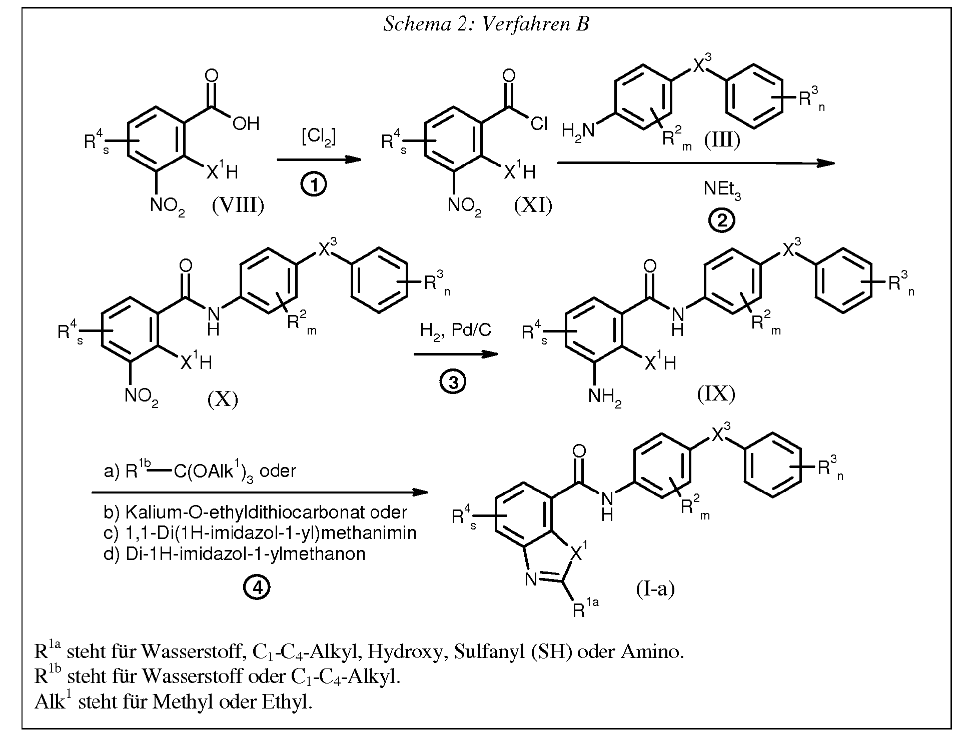 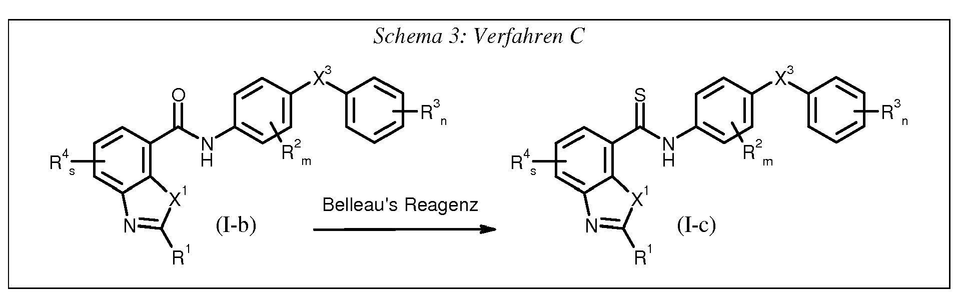 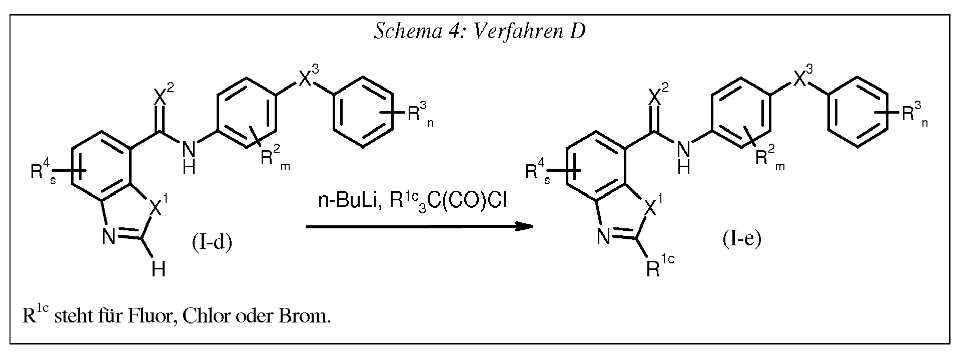 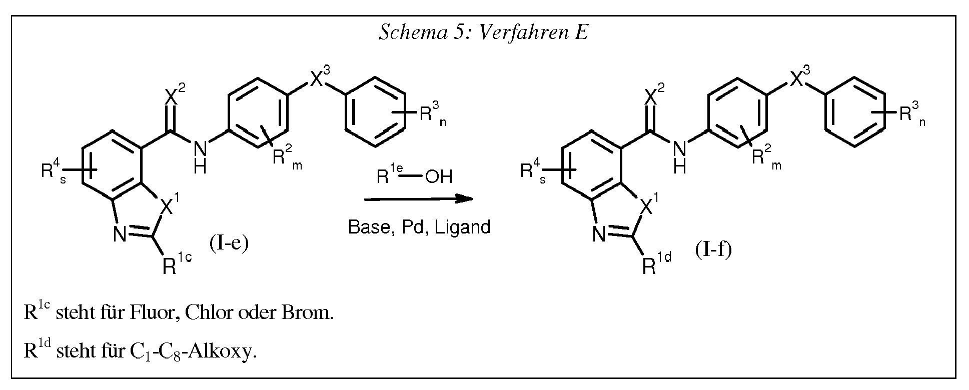 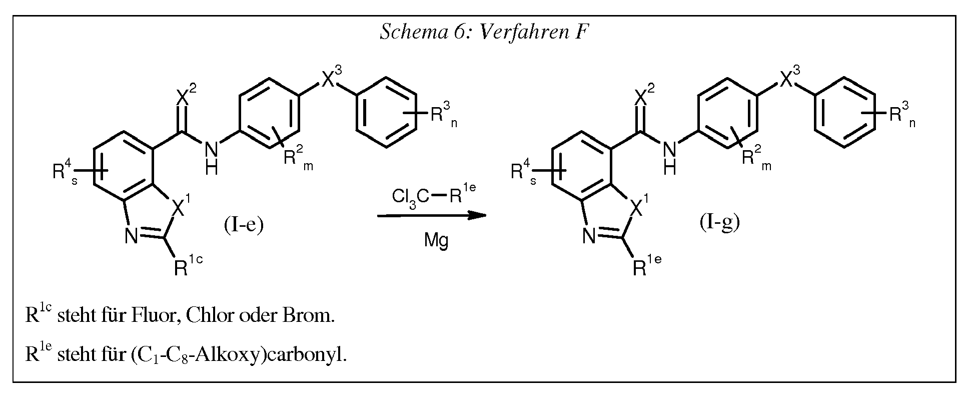 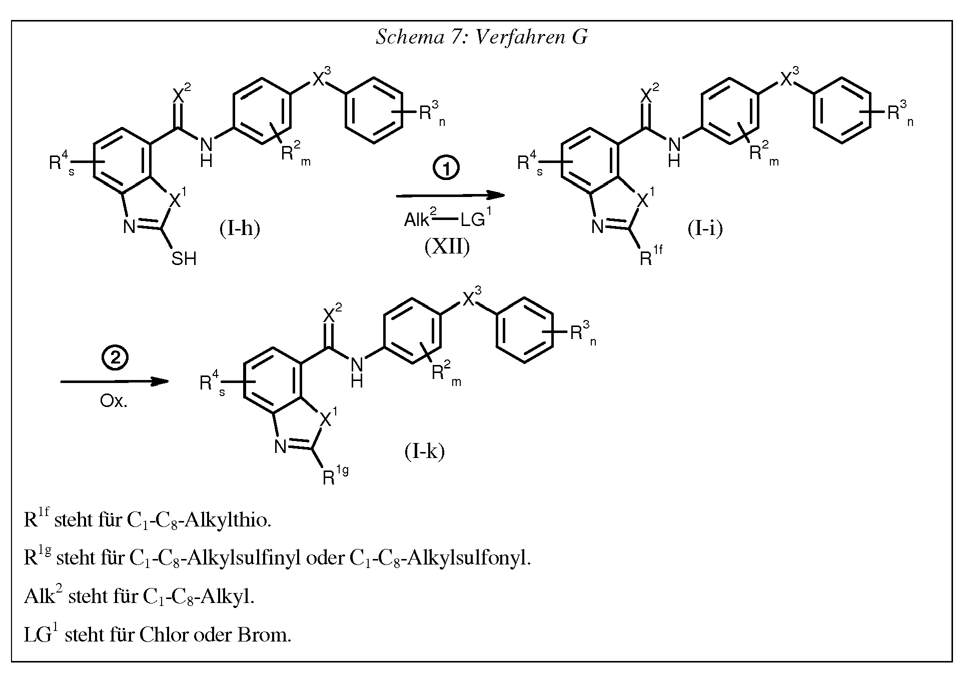 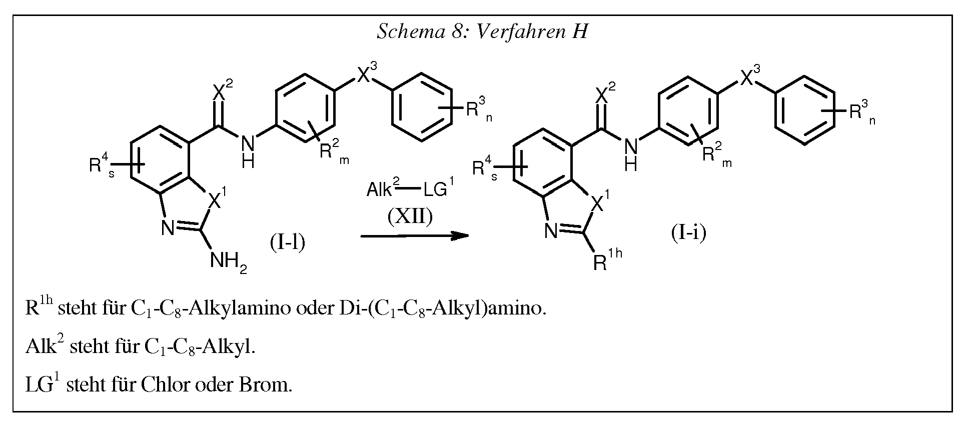 Bevorzugte Restedefinitionen der vorstehenden und nachfolgend genannten Formeln und Schemata sind bereits oben angegeben. Diese Definitionen gelten nicht nur für die Endprodukte der Formel (I) sondern auch für alle Zwischenprodukte gleichermaßen.
- R^{1a}: steht bevorzugt für Wasserstoff, Methyl, Hydroxy, Sulfanyl (SH) oder Amino.
- R^{1a}: steht besonders bevorzugt für Wasserstoff oder Sulfanyl (SH).
- R^{1a}: steht ganz besonders bevorzugt für Wasserstoff.
- R^{1b}: steht bevorzugt für Wasserstoff oder Methyl.
- R^{1b}: steht besonders bevorzugt für Wasserstoff.
- R^{1c}: steht bevorzugt für Chlor oder Brom.
- R^{1c}: steht besonders bevorzugt für Chlor.
- R^{1d}: steht bevorzugt für Methoxy oder Ethoxy.
- R^{1e}: steht bevorzugt für Methoxycarbonyl oder Ethoxycarbonyl.
- R^{1f}: steht bevorzugt für Methylthio.
- R^{1g}: steht bevorzugt für Methylsulfinyl, Ethylsulfinyl, Methylsulfonyl oder Ethylsulfonyl.
- R^{1h}: steht bevorzugt für Methylamino oder Dimethylamino.
- Alk²: steht bevorzugt für Methyl oder Ethyl.

Folgende Verbindungen der vorstehenden Schemata sind neu und damit ebenfalls Teil der vorliegenden Erfindung:

Nitrobenzoesäure-Derivate der Formel (VIII-a), 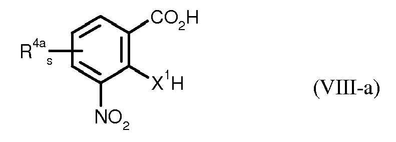 in welcher
- X¹ und s: die oben angegebenen Bedeutungen haben,
- R^{4a}: für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)-sulfinyl(C₁-C₄-alkyl), (C₁-C₄-Alkyl)sulfonyl(C₁-C₄-alkyl), Formyl, C₁-C-₈-Alkylcarbonyl, C₁-C₈-Alkylamino, Di-(C₁-C₈-alkyl)anüno, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.
- R^{4a}: steht bevorzugt für Methylthio, Ethylthio, n-Propylthio, i-Propylthio, n-, i-, s- oder t- Butylthio, Dimethylamino, Trifluormethyl oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4a}: steht besonders bevorzugt für Methoxy oder Trifluormethyl.

Aminobenzoesäure-Derivate der Formel (VII-a), 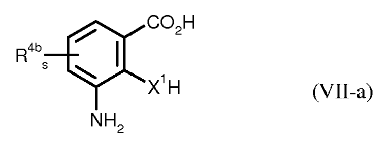 in welcher
- X¹ und s: die oben angegebenen Bedeutungen haben,
- R^{4b}: für C₁-C₈-Alkylsulfinyl, C₁-C₄-Alkoxy-C₁-C₄-alkyl, (C₁-C-₄-Alkyl)thio(C₁-C₄-alkyl), (C₁-C₄-Alkyl)-sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.
- R^{4b}: steht bevorzugt für Methylthio, Ethylthio, n-Propylthio, i-Propylthio, n-, i-, s- oder t- Butylthio, Dimethylamino, Trifluormethyl oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4b}: steht besonders bevorzugt für Methoxy oder Trifluormethyl.

Neu sind Verbindungen der Formel (VI-a), 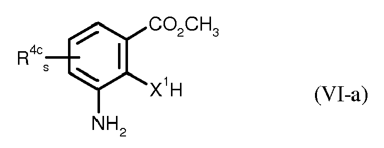 in welcher
- X¹ und s: die oben angegebenen Bedeutungen haben,
- R^{4c}: für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)-sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogen-alkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.
- R^{4c}: steht bevorzugt für Methylthio, Ethylthio, n-Propylthio, i-Propylthio, n-, i-, s- oder t- Butylthio, Dimethylamino, Trifluormethyl oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4c}: steht besonders bevorzugt für Methoxy oder Trifluormethyl.

Neu sind Benzoxa(thia)zolcarbonsäureester der Formel (V-a), 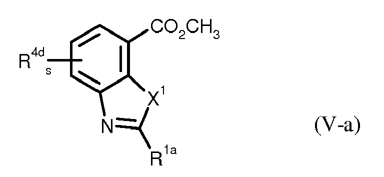 in welcher
- X¹, R^{1a} und s: die oben angegebenen Bedeutungen haben,
- R^{4d}: für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)-sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.
- R^{4d}: steht bevorzugt für Methylthio, Ethylthio, n-Propylthio, i-Propylthio, n-, i-, s- oder t- Butylthio, Dimethylamino, Trifluormethyl oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4d}: steht besonders bevorzugt für Methoxy oder Trifluormethyl.

Neu sind Verbindungen der Formel (IV-a), 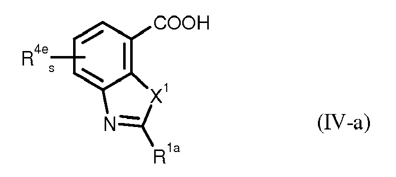 in welcher
- X¹ R^{1a}und s: die oben angegebenen Bedeutungen haben,
- R^{4e}: für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)-sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.
- R^{4e}: steht bevorzugt für Methylthio, Ethylthio, n-Propylthio, i-Propylthio, n-, i-, s- oder t- Butylthio, Dimethylamino, Trifluormethyl oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4e}: steht besonders bevorzugt für Methoxy oder Trifluormethyl.

Neu sind Verbindungen der Formel (X-a), 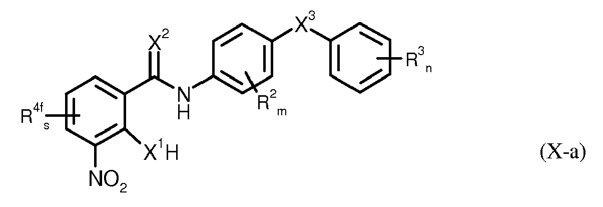 in welcher
- X¹, X², X³, R², m, R³, n und s: die oben angegebenen Bedeutungen haben,
- R^{4f}: für Halogen, Hydroxy, Cyano, Nitro, C₁-C₈-Alkyl, C₁-C₈-Alkoxy, C₁-C₈-Alkylthio, C₁-C₈-Alkylsulfinyl, C₁-C₈-Alkylsulfonyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Phenyl (welches wiederum gegebenenfalls einfach oder mehrfach, gleich oder verschieden durch Halogen, Cyano, Nitro, C₁-C₆-Alkyl, C₁-C₆-Alkoxy, C₁-C₆-Alkylthio, C₁-C₆-Halogenalkyl, C₁-C₆-Halogenalkylthio substituiert sein kann), Heteroaryl, Heterocyclyl steht.
- R^{4f}: steht bevorzugt für Methylthio, Ethylthio, n-Propylthio, i-Propylthio, n-, i-, s- oder t- Butylthio, Dimethylamino, Dimethylaminosulfonyl, Trifluormethyl, Trichlormethoxy, Trifluormethoxy oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4f}: besonders bevorzugt für Fluor, Chlor, Brom, Iod, Cyano, Nitro, Methyl, Methoxy oder Trifluormethoxy steht.

Neu sind Verbindungen der Formel (XI-a), 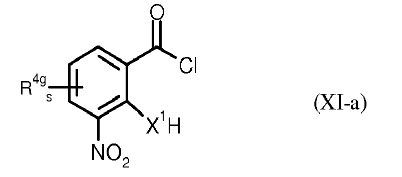 in welcher
- X¹ und s: die oben angegebenen Bedeutungen haben,
- R^{4g}: für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)-sulfinyl(C₁-C₄-alkyl), (C₁-C-₄-Alkyl)sulfonyl(C₁-C₄-alkyl), Formyl, C₁-C₈-Alkylcarbonyl, C₁-C-₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.
- R^{4g}: steht bevorzugt für Methylthio, Ethylthio, n-Propylthio, i-Propylthio, n-, i-, s- oder t- Butylthio, Dimethylamino, Trifluormethyl oder Heteroaryl (welches bevorzugt ausgewählt ist aus 1-Imidazolyl, 3-Thiazolyl, 3-Oxazolyl, 2-Pyridyl, 3-Pyridyl, 4-Pyridyl).
- R^{4g}: steht besonders bevorzugt für Methoxy oder Trifluormethyl.

### Verfahren A

Die bei der Durchführung des erfindungsgemäßen Verfahrens A in Schritt ① als Ausgangstoffe benötigten Nitrobenzoesäure-Derivate der Formel (VIII) sind teilweise neu [siehe oben Verbindungen der Formel (VIII-a)]. Sowohl die neuen als auch die literaturbeschriebenen Verbindungen lassen sich mittels bekannter Methoden herstellen [Journal of Organic Chemistry (2008) 73(22), 9121-9124, Helvetica Chimica Acta (2008) 91(8), 1435-1442, Journal of Medicinal Chemistry (2008) 51(5), 1203-1213, Tetrahedron (2007) 63(51), 12646-12654, Advanced Synthesis & Catalysis (2007) 349(17+18), 2673-2676, Chem-MedChem (2007) 2(12), 1708-1712, Heterocycles (2008) 75(6), 1425-1433, Angewandte Chemie, International Edition (2008) 47(17), 3234-3237, Chemical Biology & Drug Design (2008) 71(2), 167-172]. Gegebenenfalls wird Salicylsäure bzw. 2-Sulfanylbenzoesäure zunächst in 4-, 5- und/oder 6-Position mit R² substituiert [Revue Roumaine de Chimie (1987) 32(2), 215-21] und dann in 3-Position nitriert *[*Journal of Medicinal Chemistry (1986) 29(1), 25-9; Revue Roumaine de Chimie (1987) 32(2), 215-21; Journal of Organic Chemistry (1994) 59(15), 4297-303] oder es wird 4-, 5-, oder 6-Halogensalicylsäure in 4-, 5-und/oder 6-Position mit R² substituiert und anschließend in 3-Position nitriert.

Es ist auch möglich Nitrobenzoesäure-Derivate der Formel (VIII), in welcher R⁴ für Halogen, bevorzugt für Fluor, Chlor oder Brom, steht, nach bekannten Verfahren zu derivatisieren [Journal of Organic Chemistry (1994) 59(15), 4297-303]. Die bei der Durchführung des erfindungsgemäßen Verfahrens A in Schritt ① erhaltenen Aminobenzoesäure-Derivate der Formel (VII) können alternativ zum Verfahren A/Schritt ① teilweise auch nach folgendem Verfahren erhalten werden (für den Fall, dass X¹ für Schwefel steht): 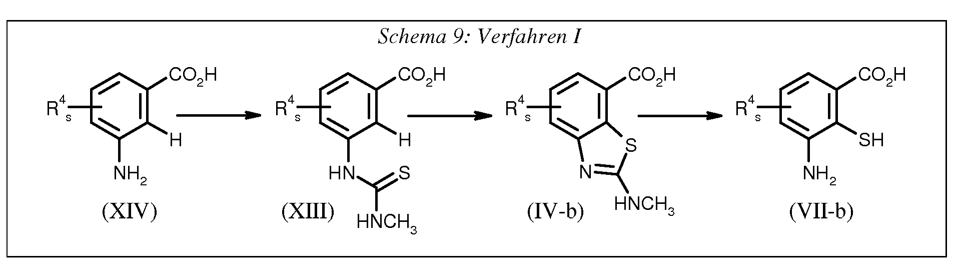 3-Aminobenzoesäure-Derivate der Formel (XIV) sind bekannt.

Es ist auch möglich, die Aminobenzoesäure-Derivate der Formel (VII) ohne Methylierung direkt weiter umzusetzen, z.B. nach Variante a) in Schritt ③. Dadurch entfällt dann auch die Freisetzung der Säure gemäß Schritt ④.

Die bei der Durchführung des erfindungsgemäßen Verfahrens A in Schritt ② erhaltenen Benzoxa(thia)zolcarbonsäureester der Formel (V) können alternativ zum Verfahren A/Schritt ② teilweise auch nach folgendem Verfahren erhalten werden (für den Fall, dass R¹ für Wasserstoff steht): 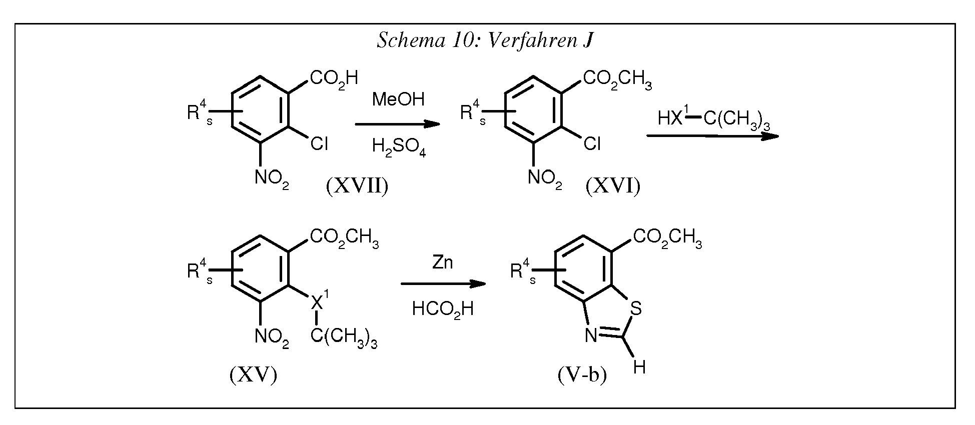 2-Chlor-3-nitrobenzoesäure-Derivate der Formel (XVII) sind bekannt oder können analog den Verbindungen der Formel (VIII) - wie oben beschrieben - erhalten werden.

### Reaktionsbedingungen für Schritt ① (Verfahren A)

Als geeignete Katalysatoren zur Durchführung der katalytischen Hydrierung kommen alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid), Nickel-Katalysatoren (z.B. reduziertes Nickel, Nickeloxid, Raney-Nickel) infrage. Vorzugsweise verwendet man jedoch Edelmetallkatalysatoren, wie beispielsweise Platin- und Palladium-Katalysatoren gegebenenfalls auf einem geeigneten Träger wie beispielsweise auf Kohlenstoff, besonders bevorzugt Pd/C.

Bei der Hydrierungsreaktion kommen jeweils alle üblichen inerten, organischen Solventien infrage. Vorzugsweise verwendbar sind Alkohole, wie Methanol oder Ethanol, Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Ester wie Essigsäuremethylester oder Essigsäureethylester; Sulfoxide, wie Dimethylsulfoxid; Sulfone, wie Sulfolan, organische Säuren wie Ameisensäure oder Essigsäure oder Wasser. Es ist auch möglich, Gemische der genannten Lösungs- und Verdünnungsmittel einzusetzen. Besonders bevorzugt verwendet man Alkohole wie Methanol oder Ethanol und Ether wie Dioxan und Tetrahydrofuran sowie deren Gemische.

Die Reaktionstemperaturen können bei der Durchführung von Schritt ① des erfindungsgemäßen Verfahrens A in einem größeren Bereich variiert werden. Im Allgemeinen arbeitet man bei Temperaturen von -78°C bis +150°C, vorzugsweise bei Temperaturen von 0°C bis +110°C.

Zur Durchführung von Schritt ① des erfindungsgemäßen Verfahrens A hydriert man Nitrobenzoesäure-Derivate der Formel (VIII) im Allgemeinen in Gegenwart von 0.1 bis 50 Gewichts-%, vorzugsweise 0.5 bis 10 Gewichts-% Übergangsmetallkatalysator. Die Reaktionsdauer beträgt 1 bis 48 Stunden. Es kann grundsätzlich unter Normaldruck gearbeitet werden. Vorzugsweise arbeitet man bei Normaldruck oder bei Drucken bis zu 15 bar in einer Wasserstoffatmosphäre.

### Reaktionsbedingungen für Schritt ② (Verfahren A)

Bei Verwendung von Diazomethan als Methylierungsmittel kommen bei der Durchführung von Schritt ② des erfindungsgemäßen Verfahrens A vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Schritt ② des erfindungsgemäßen Verfahrens A wird bei Verwendung von Diazomethan gegebenenfalls in Gegenwart eines geeigneten Säureakzeptors durchgeführt. Als solche kommen übliche anorganische oder organische Basen infrage. Hierzu gehören vorzugsweise Alkalimetallcarbonate oder -hydrogencarbonate, wie z.B. Lithiumcarbonat, Natriumcarbonat, Kaliumcarbonat, Caesiumcarbonat, Kaliumhydrogencarbonat oder Natriumhydrogencarbonat; tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU), sowie Protonenschwamm. (1,8-Bis-dimethylamino-naphthalin).

Die Reaktionstemperaturen können bei der Durchführung von Schritt ② des erfindungsgemäßen Verfahrens A in einem größeren Bereich variiert werden. Im Allgemeinen arbeitet man bei Temperaturen von -78°C bis +150°C, vorzugsweise bei Temperaturen von -78°C bis +50°C, ganz besonders bevorzugt bei 0°C bis 30°C.

Zur Durchführung von Schritt ② des erfindungsgemäßen Verfahrens A setzt man pro Mol an Verbindung der Formel (VII) im Allgemeinen 0,5 bis 20 Mol, vorzugsweise 1 bis 5 Mol an Diazomethan sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Reaktionsbedingungen für Schritt ③ (Verfahren A)

a) Bei Verwendung von R^{1b}-C(OAlk¹)₃ wird das Reagenz R^{1b}-C(OAlk¹)₃ als Lösungsmittel verwendet oder es kommen bei der Durchführung von Schritt ③ des erfindungsgemäßen Verfahrens A vorzugsweise folgende Lösungsmittel infrage: alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.
   Schritt ③ des erfindungsgemäßen Verfahrens A wird bei Verwendung von R^{1b}-C(OAlk¹)₃ gegebenenfalls in Gegenwart eines geeigneten Katalysators durchgeführt. Als solche kommen PPTS, p-TsOH, H₂SO₄ und HCl und andere inerte Säuren in Frage.
   Die Reaktionstemperaturen können bei der Durchführung von Schritt ③ des erfindungsgemäßen Verfahrens A in einem größeren Bereich variiert werden. Im Allgemeinen arbeitet man bei Temperaturen von 0°C bis +250°C, vorzugsweise bei Temperaturen von 20°C bis +150°C, ganz besonders bevorzugt bei 40°C bis 120°C.
   Zur Durchführung von Schritt ③ des erfindungsgemäßen Verfahrens A setzt man pro Mol an Verbindung der Formel (VI) im Allgemeinen 0,5 bis 1000 Mol, vorzugsweise 100 bis 1000 Mol an R^{1b}-C(OAlk¹) sowie 0,01 bis 20 Mol, vorzugsweise 0,1 bis 1 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.
b) Bei Verwendung von Kalium-O-ethyldithiocarbonat kommen bei der Durchführung von Schritt ③ des erfindungsgemäßen Verfahrens A vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.
   Die Reaktionstemperaturen können bei der Durchführung von Schritt ③ des erfindungsgemäßen Verfahrens A in einem größeren Bereich variiert werden. Im Allgemeinen arbeitet man bei Temperaturen von 0°C bis +250°C, vorzugsweise bei Temperaturen von 20°C bis +200°C, ganz besonders bevorzugt bei der Siedetemperatur des entsprechenden Lösungsmittels.
   Zur Durchführung von Schritt ③ des erfindungsgemäßen Verfahrens A setzt man pro Mol an Verbindung der Formel (VII) im Allgemeinen 0,5 bis 20 Mol, vorzugsweise 1 bis 5 Mol an Kalium-O-ethyldithiocarbonat ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.
c,d) Bei Verwendung von 1,1-Di(1H-imidazol-1-yl)methanimin oder Di -1H-imidazol-1-ylmethanon kommen bei der Durchführung von Schritt ③ des erfindungsgemäßen Verfahrens A vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.
   Die Reaktionstemperaturen können bei der Durchführung von Schritt ③ des erfindungsgemäßen Verfahrens A in einem größeren Bereich variiert werden. Im Allgemeinen arbeitet man bei Temperaturen von - 78°C bis +250°C, vorzugsweise bei Temperaturen von 40°C bis +200°C, ganz besonders bevorzugt bei der Siedetemperatur des entsprechenden Lösungsmittels.
   Zur Durchführung von Schritt ③ des erfindungsgemäßen Verfahrens A setzt man pro Mol an Verbindung der Formel (VII) im Allgemeinen 0,5 bis 20 Mol, vorzugsweise 1 bis 5 Mol an 1,1-Di(1H-imidazol-1-yl)methanimin oder Di -1H-imidazol-1-ylmethanon ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Reaktionsbedingungen für Schritt ④ (Verfahren A)

Die Freisetzung der Säure erfolgt nach üblichen Methoden (Organic Chemistry, J. Clayden, N. Greeves, S. Warren; P. Wothers, Oxford University Press 2001), z.B. durch Verseifung der Benzoxa(thia)zolcarbonsäureester der Formel (V) mit 0,5 bis 20 Mol, vorzugsweise mit 1 bis 5 Mol Lithiumhydroxid, wobei eine 0,1 bis 5 molare Lösung, vorzugsweise eine 1-2 molare wässrige Lösung verwendet wird.

### Reaktionsbedingungen für Schritt ⑤ (Verfahren A)

Die Chlorierung in Schritt ⑤ erfolgt nach üblichen Methoden. Die Umsetzung der Benzoxa(thia)zolcarbonsäuren der Formel (IV) erfolgt mit einem Chlorierungsmittel (z.B. Thionylchlorid/Oxalylchlorid) in Gegenwart eines Verdünnungsmittels (z.B. Toluol oder Methylenchlorid).

Die Reaktionstemperaturen können bei der Durchführung von Schritt ⑤ des erfindungsgemäßen Verfahrens A in einem größeren Bereich variiert werden. Im Allgemeinen arbeitet man bei Temperaturen von 0°C bis +150°C, vorzugsweise bei Temperaturen von 20°C bis zu der Siedetemperatur des entsprechenden Lösungsmittels.

Zur Durchführung von Schritt ⑤ des erfindungsgemäßen Verfahrens A setzt man pro Mol an Verbindung der Formel IV) im Allgemeinen 0,5 bis 20 Mol, vorzugsweise 1 bis 1,5 Mol an Chlorierungsmittel ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Reaktionsbedingungen für Schritt ⑥ (Verfahren A)

Als Verdünnungsmittel zur Durchführung von Schritt ⑥ des erfindungsgemäßen Verfahrens A kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Schritt ⑥ des erfindungsgemäßen Verfahrens A wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Schritt ⑥ des erfindungsgemäßen Verfahrens A wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Schritt ⑥ des erfindungsgemäßen Verfahrens A wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung von Schritt ⑥ des erfindungsgemäßen Verfahren A in einem größeren Bereich variiert werden. Im Allgemeinen arbeitet man bei Temperaturen von 0°C bis 150°C, vorzugsweise bei Temperaturen von 0°C bis 80°C.

Zur Durchführung von Schritt ⑥ des erfindungsgemäßen Verfahrens A setzt man pro Mol des Benzoxa(thia)zolcarbonsäurechlorids der Formel (II) im Allgemeinen 0,8 bis 15 Mol, vorzugsweise 0,8 bis 8 Mol an Amin der Formel (III) ein.

### Verfahren B

Die bei der Durchführung des erfindungsgemäßen Verfahrens B in Schritt ① als Ausgangstoffe benötigten Nitrobenzoesäure-Derivate der Formel (VIII) sind bereits oben bei Verfahren A beschrieben worden.

### Reaktionsbedingungen für Schritt ① (Verfahren B)

Die Chlorierung in Schritt ① erfolgt nach üblichen Methoden. Die Umsetzung der Nitrobenzoesäure-Derivate der Formel (VIII) erfolgt mit einem Chlorierungsmittel (z.B. Thionylchlorid/Oxalylchlorid) in Gegenwart eines Verdünnungsmittels (z.B. Toluol oder Methylenchlorid).

Die Reaktionstemperaturen können bei der Durchführung von Schritt ① des erfindungsgemäßen Verfahrens B in einem größeren Bereich variiert werden. Im Allgemeinen arbeitet man bei Temperaturen von 0°C bis +150°C, vorzugsweise bei Temperaturen von 20°C bis zu der Siedetemperatur des entsprechenden Lösungsmittels.

Zur Durchführung von Schritt ① des erfindungsgemäßen Verfahrens B setzt man pro Mol an Verbindung der Formel (VIII) im Allgemeinen 0,5 bis 20 Mol, vorzugsweise 1 bis 1,5 Mol an Chlorierungsmittel ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Reaktionsbedingungen für Schritt ② (Verfahren B)

Als Verdünnungsmittel zur Durchführung von Schritt ② des erfindungsgemäßen Verfahrens B kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Schritt ② des erfindungsgemäßen Verfahrens B wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Schritt ② des erfindungsgemäßen Verfahrens B wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodümid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Schritt ② des erfindungsgemäßen Verfahrens B wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung von Schritt ② des erfindungsgemäßen Verfahren B in einem größeren Bereich variiert werden. Im Allgemeinen arbeitet man bei Temperaturen von 0°C bis 150°C, vorzugsweise bei Temperaturen von 0°C bis 80°C.

Zur Durchführung von Schritt ② des erfindungsgemäßen Verfahrens B setzt man pro Mol des Nitrobenzoesäurechlorids der Formel (XI) im Allgemeinen 0,8 bis 15 Mol, vorzugsweise 0,8 bis 8 Mol an Amin der Formel (III) ein.

### Reaktionsbedingungen für Schritt ③ (Verfahren B)

Als geeignete Katalysatoren zur Durchführung der katalytischen Hydrierung kommen alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid), Nickel-Katalysatoren (z.B. reduziertes Nickel, Nickeloxid, Raney-Nickel) infrage. Vorzugsweise verwendet man jedoch Edelmetallkatalysatoren, wie beispielsweise Platin- und Palladium-Katalysatoren gegebenenfalls auf einem geeigneten Träger wie beispielsweise auf Kohlenstoff, besonders bevorzugt Pd/C.

Bei der Hydrierungsreaktion kommen jeweils alle üblichen inerten, organischen Solventien infrage. Vorzugsweise verwendbar sind Alkohole, wie Methanol oder Ethanol, Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Ester wie Essigsäuremethylester oder Essigsäureethylester; Sulfoxide, wie Dimethylsulfoxid; Sulfone, wie Sulfolan, organische Säuren wie Ameisensäure oder Essigsäure oder Wasser. Es ist auch möglich, Gemische der genannten Lösung- und Verdünnungsmittel einzusetzen. Besonders bevorzugt verwendet man Alkohole wie Methanol oder Ethanol und Ether wie Dioxan und Tetrahydrofuran sowie deren Gemische.

Die Reaktionstemperaturen können bei der Durchführung von Schritt ③ des erfindungsgemäßen Verfahrens A in einem größeren Bereich variiert werden. Im Allgemeinen arbeitet man bei Temperaturen von -78°C bis +150°C, vorzugsweise bei Temperaturen von 0°C bis +110°C.

Zur Durchführung von Schritt ③ des erfindungsgemäßen Verfahrens A hydriert man Nitrobenzoesäureamide der Formel (X) im Allgemeinen in Gegenwart von 0.1 bis 50 Gewichts-%, vorzugsweise 0.5 bis 10 Gewichts-% Übergangsmetallkatalysator. Die Reaktionsdauer beträgt 1 bis 48 Stunden. Es kann grundsätzlich unter Normaldruck gearbeitet werden. Vorzugsweise arbeitet man bei Normaldruck oder bei Drucken bis zu 15 bar in einer Wasserstoffatmosphäre.

### Reaktionsbedingungen für Schritt ④ (Verfahren B)

a) Bei Verwendung von R^{1b}-C(OAlk¹)₃ wird das Reagenz R^{1b}-C(OAlk¹)₃ als Lösungsmittel verwendet oder es kommen bei der Durchführung von Schritt ④ des erfindungsgemäßen Verfahrens B vorzugsweise folgende Lösungsmittel infrage: alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.
   Schritt ④ des erfindungsgemäßen Verfahrens B wird bei Verwendung von R^{1b}-C(OAlk¹)₃ gegebenenfalls in Gegenwart eines geeigneten Katalysators durchgeführt. Als solche kommen PPTS, p-TsOH, H₂SO₄ und HCl und andere inerte Säuren in Frage.
   Die Reaktionstemperaturen können bei der Durchführung von Schritt ④ des erfindungsgemäßen Verfahrens B in einem größeren Bereich variiert werden. Im Allgemeinen arbeitet man bei Temperaturen von 0°C bis +250°C, vorzugsweise bei Temperaturen von 20°C bis +150°C, ganz besonders bevorzugt bei 40°C bis 120°C.
   Zur Durchführung von Schritt ④ des erfindungsgemäßen Verfahrens B setzt man pro Mol an Verbindung der Formel (IX) im Allgemeinen 0,5 bis 1000 Mol, vorzugsweise 100 bis 1000 Mol an R^{1b}-C(OAlk¹) sowie 0,01 bis 20 Mol, vorzugsweise 0,1 bis 1 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.
b) Bei Verwendung von Kalium-O-ethyldithiocarbonat kommen bei der Durchführung von Schritt ④ des erfindungsgemäßen Verfahrens B vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.
   Die Reaktionstemperaturen können bei der Durchführung von Schritt ④ des erfindungsgemäßen Verfahrens B in einem größeren Bereich variiert werden. Im Allgemeinen arbeitet man bei Temperaturen von 0°C bis +250°C, vorzugsweise bei Temperaturen von 20°C bis +200°C, ganz besonders bevorzugt bei der Siedetemperatur des entsprechenden Lösungsmittels.
   Zur Durchführung von Schritt ④ des erfindungsgemäßen Verfahrens B setzt man pro Mol an Verbindung der Formel (IX) im Allgemeinen 0,5 bis 20 Mol, vorzugsweise 1 bis 5 Mol an Kalium-O-ethyldithiocarbonat ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.
c,d) Bei Verwendung von 1,1-Di(1H-inüdazol-1-yl)methanimin oder Di-1H-imidazol-1-ylmethanon kommen bei der Durchführung von Schritt ④ des erfindungsgemäßen Verfahrens B vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.
   Die Reaktionstemperaturen können bei der Durchführung von Schritt ④ des erfindungsgemäßen Verfahrens B in einem größeren Bereich variiert werden. Im Allgemeinen arbeitet man bei Temperaturen von - 78°C bis +250°C, vorzugsweise bei Temperaturen von 40°C bis +200°C, ganz besonders bevorzugt bei der Siedetemperatur des entsprechenden Lösungsmittels.
   Zur Durchführung von Schritt ④ des erfindungsgemäßen Verfahrens B setzt man pro Mol an Verbindung der Formel (IX) im Allgemeinen 0,5 bis 20 Mol, vorzugsweise 1 bis 5 Mol an 1,1-Di(1H-imidazol-1-yl)methanimin oder Di -1H-imidazol-1-ylmethanon ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren C

Die bei der Durchführung des Verfahrens C als Ausgangsstoffe benötigten Benzoxa(thia)zole der Formel (I-b) können je nach Bedeutung der Reste X¹, R¹, R² m und R³ nach den Verfahren A, B, D, E, F, G oder H (wobei X² dann jeweils für Sauerstoff steht) erhalten werden.

Belleau's Reagenz ist 2,4-Bis(4-phenoxyphenyl)-1,3-dithia-2,4-diphosphetan-2,4-disulfid.

Bei Verwendung von Belleau's Reagenz als Verschwefelungsreagenz kommen bei Verfahrens C vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung des erfindungsgemäßen Verfahrens C in einem größeren Bereich variiert werden. Im Allgemeinen arbeitet man bei Temperaturen von -78°C bis +150°C, vorzugsweise bei Temperaturen von -78°C bis +50°C, ganz besonders bevorzugt bei 0°C bis 30°C.

Zur Durchführung des Verfahrens C setzt man pro Mol an Verbindung der Formel (I-b) im Allgemeinen 0,5 bis 20 Mol, vorzugsweise 1 bis 5 Mol an Belleau's Reagenz ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren D

Die bei der Durchführung des Verfahrens D als Ausgangsstoffe benötigten Benzoxa(thia)zole der Formel (I-d) können je nach Bedeutung der Reste X¹, X², R², m und R³ nach den Verfahren A, B oder C erhalten werden.

Bei Verwendung von *n*-BuLi und R^{1c}₃C(CO)Cl kommen bei Verfahren D vorzugsweise folgende Lösungsmittel infrage: Alkohole, wie z.B. Methanol, Ethanol oder Isopropanol; aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung von Verfahren D in einem größeren Bereich variiert werden. Im Allgemeinen arbeitet man bei Temperaturen von -78°C bis +150°C, vorzugsweise bei Temperaturen von -78°C bis +50°C, ganz besonders bevorzugt bei -78°C bis 20°C.

Zur Durchführung des Verfahrens D setzt man pro Mol an Verbindung der Formel (I-d) im Allgemeinen 0,5 bis 20 Mol, vorzugsweise 0,9 bis 1,5 Mol an *n*-BuLi und 0,5 bis 20 Mol, vorzugsweise 1 bis 5 Mol an R^{1c}₃C(CO)Cl ein.

Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren E

Die bei der Durchführung des Verfahrens E als Ausgangsstoffe benötigten Benzoxa(thia)zole der Formel (I-e) können nach Verfahren D erhalten werden.

Bei Verwendung von einer Base, einem Alkohol, Palladium und einem Liganden als Alkoxygenierungsreagenzien kommen bei Verfahrens E vorzugsweise folgende Lösungsmittel infrage: aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung von Verfahren E in einem größeren Bereich variiert werden. Im Allgemeinen arbeitet man bei Temperaturen von -78°C bis +150°C, vorzugsweise bei Temperaturen von -78°C bis +50°C, ganz besonders bevorzugt bei 0°C bis 30°C.

Zur Durchführung des Verfahrens E setzt man pro Mol an Verbindung der Formel (I-d) im Allgemeinen 0,5 bis 20 Mol, vorzugsweise 1 bis 5 Mol an Base, 0,5 bis 20 Mol, vorzugsweise 1 bis 5 Mol an Alkohol, 0,01 bis 1 Mol, vorzugsweise 0,1 bis 1 Mol Palladium und 0,5 bis 20 Mol, vorzugsweise 1 bis 5 Mol an Ligand ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren F

Die bei der Durchführung des Verfahrens F als Ausgangsstoffe benötigten Benzoxa(thia)zole der Formel (I-e) können nach Verfahren D erhalten werden.

Bei Verwendung von Magnesium und Cl₃CR^{1e} als Alkoxycarbonylierungsreagenzien kommen bei Verfahren E vorzugsweise folgende Lösungsmittel infrage: aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung von Verfahren F in einem größeren Bereich variiert werden. Im Allgemeinen arbeitet man bei Temperaturen von -78°C bis +150°C, vorzugsweise bei Temperaturen von -78°C bis +50°C, ganz besonders bevorzugt bei 0°C bis 30°C.

Zur Durchführung des Verfahrens F setzt man pro Mol an Verbindung der Formel (I-f) im Allgemeinen 0,5 bis 20 Mol, vorzugsweise 1 bis 5 Mol an Magnesium, 0,5 bis 20 Mol, vorzugsweise 1 bis 5 Mol an Cl₃CR^{1e} ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren G

Die bei der Durchführung des Verfahrens F als Ausgangsstoffe benötigten Benzoxa(thia)zole der Formel (I-h) können z.B. nach Verfahren A erhalten werden.

Die bei der Durchführung des Verfahrens G weiterhin als Ausgangsstoffe benötigten Alkylchloride bzw. - bromide der Formel (XII) sind bekannt.

### Reaktionsbedingungen für Schritt ① (Verfahren G)

Bei Verwendung von Halogenalkan als Alkylierungsmittel kommen bei der Durchführung von Schritt ① des erfindungsgemäßen Verfahrens G vorzugsweise folgende Lösungsmittel infrage: aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung von Schritt ① des erfindungsgemäßen Verfahrens G in einem größeren Bereich variiert werden. Im Allgemeinen arbeitet man bei Temperaturen von - 78°C bis +150°C, vorzugsweise bei Temperaturen von -78°C bis +50°C, ganz besonders bevorzugt bei 0°C bis 30°C.

Zur Durchführung von Schritt ① des erfindungsgemäßen Verfahrens G setzt man pro Mol an Verbindung der Formel (I-h) im Allgemeinen 0,5 bis 20 Mol, vorzugsweise 1 bis 5 Mol an Halogenalkan sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Reaktionsbedingungen für Schritt ② (Verfahren G)

Bei Verwendung von Diazomethan als Oxidationsmittel kommen bei der Durchführung von Schritt ② des erfindungsgemäßen Verfahrens G vorzugsweise folgende Lösungsmittel infrage: aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; halogenierte Kohlenwasserstoffe, wie Chlorbenzol, Dichlorbenzol, Trichlorbenzol, Chlortoluol, Dichlormethan, Chloroform, Tetrachlormethan, 1,2-Dichlorethan, Trichlorethan, Tetrachlorethan oder Tetrachlorethylen; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung von Schritt ② des erfindungsgemäßen Verfahrens G an einem größeren Bereich variiert werden. Im Allgemeinen arbeitet man bei Temperaturen von - 78°C bis +150°C, vorzugsweise bei Temperaturen von -78°C bis +50°C, ganz besonders bevorzugt bei 0°C bis 30°C.

Zur Durchführung von Schritt ② des erfindungsgemäßen Verfahrens G setzt man pro Mol an Verbindung der Formel (I-i) im Allgemeinen 0,5 bis 20 Mol, vorzugsweise 1 bis 5 Mol an ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

### Verfahren H

Die bei der Durchführung des Verfahrens H als Ausgangsstoffe benötigten Benzoxa(thia)zole der Formel (I-1) können z.B. nach Verfahren A erhalten werden.

Bei Verwendung von Halogenalkan als Alkylierungsmittel kommen bei der Durchführung des erfindungsgemäßen Verfahrens H vorzugsweise folgende Lösungsmittel infrage: aliphatische, alicyclische oder aromatische Kohlenwasserstoffe, wie z.B. Petrolether, Pentan, Hexan, Heptan, Oktan, Nonan und technische Kohlenwasserstoffe; beispielsweise so genannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Benzol, Toluol, Xylol oder Decalin; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Ester, wie Essigsäuremethylester oder Essigsäureethylester; Ketone, wie Aceton, Butanon, Methyl-isobutylketon oder Cyclohexanon; Nitrile, wie Acetonitril, Propionitril, n- oder i-Butyronitril oder Benzonitril, m-Chlorbenzonitril. Es ist auch möglich, Gemische der genannten Lösungs- und Verdünnungsmittel einzusetzen.

Die Reaktionstemperaturen können bei der Durchführung des Verfahrens H in einem größeren Bereich variiert werden. Im Allgemeinen arbeitet man bei Temperaturen von -78°C bis +150°C, vorzugsweise bei Temperaturen von -78°C bis +50°C, ganz besonders bevorzugt bei 0°C bis 30°C.

Zur Durchführung von Verfahrens H setzt man pro Mol an Verbindung der Formel (I-1) im Allgemeinen 0,5 bis 20 Mol, vorzugsweise 1 bis 5 Mol an Halogenalkan sowie 0 bis 20 Mol, vorzugsweise 1 bis 5 Mol an Säureakzeptor ein. Die Reaktionsdauer beträgt 1 bis 48 Stunden. Die Reaktionsdurchführung erfolgt bevorzugt unter einer Schutzgasatmosphäre wie Stickstoff oder Argon. Die Aufarbeitung erfolgt nach üblichen Methoden.

Bei allen erfindungsgemäßen Verfahren wird im Allgemeinen (wenn nicht anders angegeben) unter Normaldruck gearbeitet. Es ist jedoch auch möglich, unter erhöhtem oder vermindertem Druck - im Allgemeinen zwischen 0,1 bar und 10 bar - zu arbeiten.

Die vorliegende Erfindung betrifft weiterhin ein Mittel, zum Bekämpfen von unerwünschten Mikroorganismen, umfassend die erfindungsgemäßen Wirkstoffe. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, **dadurch gekennzeichnet, dass** man die erfindungsgemäßen Wirkstoffe auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobemsteinsäureestem, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Im Allgemeinen enthalten die erfindungsgemäßen Mittel und Formulierungen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl dispergierbare Pulver, Öl mischbare fließfähige Konzentrate, Öl mischbare Flüssigkeiten, Schäume, Pasten, Pestizid ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Mikroorganismen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringem. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus oder Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke mikrobizide Wirkung auf und können zur Bekämpfung von unerwünschten Mikroorganismen, wie Pilzen und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), Ri*besioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffe auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphomährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und - abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Eltemlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Bamase selektiv in den Tapetumzellen in den Staubblättem exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmem zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_CrickmoreBt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter
   http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekommorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpftanzen mit einer erhöhten Expression der Saccharosephosphatsynthase;
d) Pflanzen wie Baumwollpftanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylhamstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen und Insekten, eingesetzt werden.

Weiter können die erfindungsgemäßen Verbindungen allein oder in Kombinationen mit anderen Wirkstoffen als Antifouling-Mittel eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geemtetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Altemaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola; Ähren- und Rispenerkrankungen (inklusive Maiskolben), hervorgerufen durch z.B. Altemaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Altemaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Darüber hinaus weisen die erfindungsgemäßen Wirkstoffeauch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe können daher sowohl in medizinischen als auch in nicht-medizinischen Anwendungen eingesetzt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp.
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Omithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp.
Aus der Ordnung der Chilopoda z.B. Geophilus spp., Scutigera spp.
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnostema consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp.
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuellebomi, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.
Weiterhin lassen sich Protozoen, wie Eimeria, bekämpfen.
Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.
Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Cameocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., E-rythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.
Aus der Ordnung der Hymenoptera z.B. Diprion spp., Hoplocampa spp., Lasius spp., Monomorium pharaonis, Vespa spp.
Aus der Ordnung der Isopoda z.B. Armadillidium vulgare, Oniscus asellus, Porcellio scaber.
Aus der Ordnung der Isoptera z.B. Reticulitermes spp., Odontotermes spp.
Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.
Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.
Aus der Ordnung der Siphonaptera z.B. Ceratophyllus spp., Xenopsylla cheopis.
Aus der Ordnung der Symphyla z.B. Scutigerella immaculata.
Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.
Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die aufgeführten Pflanzen können besonders vorteilhaft erfindungsgemäß mit den Verbindungen der allgemeinen Formel (I) den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Erfindung wird durch die folgenden Beispiele veranschaulicht. Die Erfindung ist jedoch nicht auf die Beispiele limitiert.

### Herstellungsbeispiele

### Herstellung von Verbindung Nr. 4

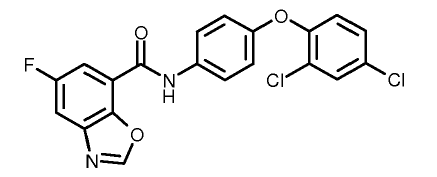

4-(2,4-Dichlorphenoxy)anilin (III-1) (210.451 mg, 0.828 mmol) wird in Dichlormethan (8 mL) und Triethylamin (0.344 mL, 2.485 mmol) gelöst und dann mit dem 1,3-Benzoxazol-5-fluor-7-carbonsäurechlorid (II-1) versetzt. Nach 10 Minuten werden alle flüchtigen Komponenten abdestilliert. Dann wird der Rückstand in Dichlormethan (15 mL) gelöst und zweimal mit gesättigter, wässriger Natriumchloridlösung gewaschen. Dann wird die organische Phase mit Natriumsulfat getrocknet und der Feststoff abfiltriert. Anschließend wird das Lösungsmittel abdestilliert. Man erhält das Produkt N-[4-(2,4-Dichlorphenoxy)phenyl]-1,3-benzoxazol-7-carboxamid (330 mg, 0.791 mmol, 96 % der Theorie) als farblosen Feststoff.

### Herstellung von Verbindung Nr. II-1

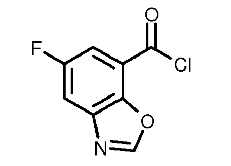

1,3-Benzoxazol-5-fluor-7-carbonsäure (IV-1) (150 mg, 0.828 mmol) werden in Dichlormethan (8 mL) gelöst. Dann wird Oxalylchlorid (0.076 mL, 0.870 mmol) und ein Tropfen DMF zugegeben. Das Reaktionsgemisch wird für 4 Stunden bei Raumtemperatur gerührt, wodurch man 1,3-Benzoxazol-5-fluor-7-carbonylchlorid (II-1) erhält. Das Reaktionsgemisch wird dann direkt in die nächste Reaktion eingesetzt.

### Herstellung von Verbindung Nr. IV-1

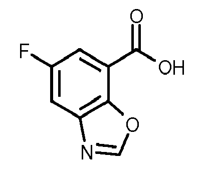

3-Amino-5-fluor-2-hydroxybenzolcarbonsäure (VII-1) (10.25g, 59.90 mmol) wird zusammen mit Trimethylorthoformat (300 mL) für 3 Stunden bei 100 °C erhitzt. Dann wird das Trimethylorthoformat abdestilliert und man erhält das Produkt 1,3-Benzoxazol-5-fluor-7-carbonsäure (10.60 g, 58.52 mmol, 59 % der Theorie) als farblosen Feststoff.

### Herstellung von Verbindung Nr. VII-1

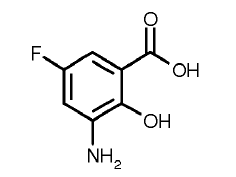

2-Nitro-5-fluorsalicylsäure (VIII-1) (16.40 g, 81.55 mmol) werden in Tetrahydrofuran (100 mL) gelöst und mit Pd/C (10 % Pd, 4.34 g, 4.08 mmol) versetzt. Unter Wasserstoffdruck (20 bar) wird das Reaktionsgemisch für 1 Stunde gerührt. Dann wird das Reaktionsgemisch mit Aktivkohle (7 g) versetzt. Der Feststoff wird abgesaugt und das Lösungsmittel am Rotationsverdampfer entfernt. Man erhält das Produkt 3-Amino-5-fluor-2-hydroxybenzolcarbonsäure (10.23 g, 59.78mmol, 70 % der Theorie) als farblosen Feststoff.

### Herstellung von Verbindung Nr. VIII-1

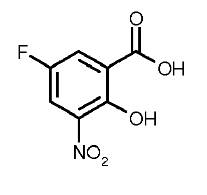

5-Fluorsalicylsäure (15 g, 96.09 mmol) werden in Essigsäure (70 mL) gelöst und dann wird eine Lösung von Salpetersäure (4.43 mL, 105.70 mmol) in Essigsäure (30 mL) zu dem Reaktionsgemisch tropfenweise dazugegeben. Anschließend wird das Reaktionsgemisch für weitere 2 Stunden gerührt. Dann wird das Reaktionsgemisch auf Eis gegeben und gerührt, bis es schmilzt. Der Feststoff wird abfiltriert und getrocknet. Man erhält das Produkt 5-Fluor-2-nitrosalicylsäure (11.5 g, 57.19 mmol, 51 % der Theorie) als oranger Feststoff.

Analog den vorangehenden Beispielen sowie entsprechend den allgemeinen Beschreibungen der erfindungsgemäßen Verfahren können die in der folgenden Tabelle 1 genannten Verbindungen der Formel (I) erhalten werden.

**Tabelle 1**

| | | | | | | | |
|---|---|---|---|---|---|---|---|
| 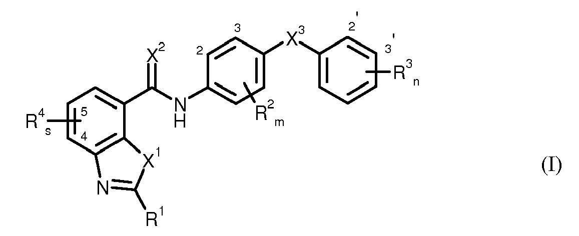 | | | | | | | |

| Nr. | X¹ | X² | X³ | R¹ | R²ₘ | R³ₙ | R⁴ₛ |
|---|---|---|---|---|---|---|---|
| 1 | O | O | O | H | - | 4'-CF₃ | 5-Cl |
| 2 | O | O | O | H | - | 2',4'-Cl₂ | 5-C1 |
| 3 | O | O | O | H | - | 4'-CF₃ | 5-F |
| 4 | O | O | O | H | - | 2',4'-Cl₂ | 5-F |
| 5 | O | O | O | H | - | 2',4'-Cl₂ | 4-CH=CH-CH=CH-5 |
| 6 | O | O | O | H | - | 4'-CF₃ | 4-CH=CH-CH=CH-5 |

**Tabelle 2: Physikalische Daten**

| Nr. | Physikalische Datena) |
|---|---|
| 1 | δ (DMSO-d6) = 7.13-7.20 (m, 4H), 7.70 (d, 2H), 7.82 (dd, 2H), 7.88 (d, 1H), 8.11 (d, 1H), 8.91 (s, 1H), 10.41 (s, 1H); LC-MS: m/z = 433 [M+H]+ |
| 2 | δ (DMSO-d6) = 7.02 (dd, 2H), 7.08 (d, 1H), 7.42 (dd, 1H), 7.68 (d, 1H), 7.75 (dd, 2H), 7.87 (d, 1H), 8.10 (d, 1H), 8.90 (s, 1H), 10.36 (s, 1H) ;LC-MS: m/z = 434 [M+H]+ |
| 3 | δ (DMSO-d6) = 7.12-7.19 (m, 4H), 7.67-7.73 (m, 3H) 7.81 (dd, 1H), 7.82 (d, 1H), 7.88 (dd, 1H), 8.90 (s, 1H), 10.38 (s, 1H); LC-MS: m/z = 417 [M+H]+ |
| 4 | δ (DMSO-d6) = 7.0 (m, 2H), 7.1 (d, 1H), 7.4 (dd, 1H), 7.7 (m, 2H), 7.8 (m, 2H), 7.9 (dd, 1H), 8.9 (s, 1H), 10.35 (s, 1H) LC-MS: m/z = 417 [M]+ |
| 5 | δ (DMSO-d6) = 7.01-7.12 (m, 4H), 7.38-7.43 (dd, 1H), 7.65-7.73 (m, 2H), 7.80 - 7.88 (m, 2H), 8.23 (d, 1H), 8.46 (s, 1H), 8.48 (d, 1H), 8.93 (s, 1H), 10.42 (s, 1H); LC-MS: m/z = 450 [M+H]+ |
| 6 | δ (DMSO-d6) = 6.99-7.12 (m, 1H), 7.12-7.22 (m, 4H), 7.65-7.75 (m, 2H), 7.82 (dt, 1H), 7.87 (ddd, 1H), 7.88 (d, 1H), 8.25 (d, 1H), 8.45 (s, 1H), 8.47 (d, 1H), 8.94 (s, 1H), 10.47 (s, 1H); LC-MS: m/z = 449 [M+H]+ |

### Anwendungsbeispiete

### Beispiel A

### Sphaerotheca-Test (Gurke) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Sphaerotheca fuliginea* inokuliert. Anschließend werden die Pflanzen in einem Gewächshaus bei 70 % relativer Luftfeuchtigkeit und einer Temperatur von 23 °C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 1, 2 und 3 bei einer Konzentration an Wirkstoff von 500 ppm einen Wirkungsgrad von 70 % oder mehr.

### Beispiel B

### Pyrenophora teres-Test (Gerste) / protektiv

| | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gerstenpflanzen mit der Wirkstoffzubereitung in der angegebenen Aufwandmenge. 1 Tag nach der Behandlung werden die Pflanzen mit einer wässrigen Sporensuspension von *Pyrenophora teres* inokuliert und verbleiben dann 48 h bei 100 % relativer Luftfeuchte und 22 °C. Anschließend werden die Pflanzen in einem Gewächshaus bei 80 % relativer Luftfeuchtigkeit und einer Temperatur von 20 °C aufgestellt.

7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen 1, 2 und 3 bei einer Konzentration an Wirkstoff von 500 ppm einen Wirkungsgrad von 70 % oder mehr.

### Beispiel C

### Spodoptera frugiperda-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z.B. die erfindungsgemäßen Verbindungen 1 und 4 eine Wirksamkeit von 80 % oder mehr bei einer Aufwandmenge von 500 g/ha.

### Beispiel D

### Phaedon cochleariae-Test (Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die erfindungsgemäßen Verbindungen 1 und 4 eine Wirksamkeit von 80 % oder mehr bei einer Aufwandmenge von 500 g/ha.

## Patentansprüche

1. Subsituierte Benzoxa(thia)zole der Formel (I) 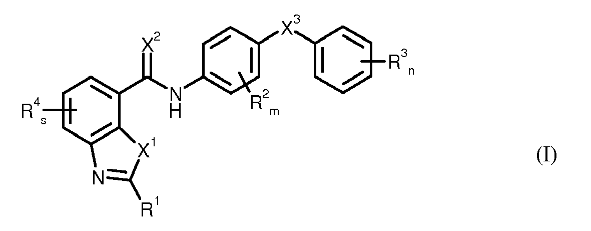 in welcher
X¹ für Sauerstoff oder Schwefel steht,
X² für Sauerstoff oder Schwefel steht,
X³ für Sauerstoff oder Schwefel steht,
R¹ für Wasserstoff, Halogen, C₁-C₄-Alkyl, Hydroxy, Sulfanyl (SH), C₁-C₈-Alkoxy, (C₁-C₈-Alkoxy)carbonyl, Amino, C₁-C₈-Alkylamino, Di-(C₁-C₈-Alkyl)amino, C₁-C₈-Alkylthio, C₁-C₈-Alkylsulfinyl oder C₁-C₈-Alkylsulfonyl steht,
R² für Halogen, Hydroxy, Cyano, Nitro, C₁-C₈-Alkyl, C₁-C₈-Alkoxy, C₁-C₈-Alkylthio, C₁-C₈-Alkylsulfinyl, C₁-C₈-Alkylsulfonyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl steht,
m für 0, 1, 2, 3 oder 4 steht, wobei R² gleich oder verschieden sein kann, wenn m für 2, 3 oder 4 steht,
R³ für Halogen, Hydroxy, Cyano, Nitro, C₁-C₈-Alkyl, C₁-C₈-Alkoxy, C₁-C₈-Alkylthio, C₁-C₈-Alkylsulfinyl, C₁-C₈-Alkylsulfonyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl steht,
n für 0, 1, 2, 3, 4 oder 5 steht, wobei R³ gleich oder verschieden sein kann, wenn n für 2, 3, 4 oder 5 steht,
R⁴ für Halogen, Hydroxy, Cyano, Nitro, C₁-C₈-Alkyl, C₁-C₈-Alkoxy, C₁-C₈-Alkylthio, C₁-C₈-Alkylsulfinyl, C₁-C₈-Alkylsulfonyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C-₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Phenyl (welches wiederum gegebenenfalls einfach oder mehrfach, gleich oder verschieden durch Halogen, Cyano, Nitro, C₁-C₆-Alkyl, C₁-C₆-Alkoxy, C₁-C₆-Alkylthio, C₁-C₆-Halogenalkyl, C₁-C₆-Halogenalkylthio substituiert sein kann), Heteroaryl, Heterocyclyl steht,
s für 1 oder 2 steht, wobei zwei benachbarte Reste R² für C₄-Alkenylen stehen, wenn s für 2 steht,
sowie deren agrochemisch wirksamen Salze.

2. Substituierte Benzoxa(thia)zole der Formel (I) gemäß Anspruch 1, in welcher
X¹ für Sauerstoff steht,
X² für Sauerstoff steht,
X³ für Sauerstoff steht,
R¹ für Wasserstoff steht,
R² für Chlor, Fluor, Brom, Methyl, Nitro, Cyano, Methoxy, Trifluormethyl steht,
m für 0 oder 1 steht,
R³ für Chlor, Fluor, Brom, Methyl, Nitro, Cyano, Methoxy, Trifluormethyl steht,
n für 0 oder 1 steht,
R⁴ für Fluor, Chlor, Brom, Iod, Cyano, Nitro, Methyl, Methoxy oder Trifluormethoxy steht,
s für 1 steht.

3. Verfahren zur Bekämpfung von pflanzenpathogenen Schadpilzen, **dadurch gekennzeichnet, dass** man Verbindungen der Formel (I) gemäß Anspruch 1 oder 2, auf die pflanzenpathogenen Schadpilze und/oder deren Lebensraum ausbringt.

4. Mittel zur Bekämpfung von pflanzenpathogenen Schadpilzen, **gekennzeichnet durch** einen Gehalt an mindestens einer Verbindung der Formel (I) gemäß Anspruch 1 oder 2, neben Streckmitteln und/oder oberflächenaktiven Stoffen.

5. Verwendung von substituierte Benzoxa(thia)zole der Formel (I), gemäß Anspruch 1 oder 2 zur Bekämpfung von pflanzenpathogenen Schadpilzen.

6. Verfahren zur Herstellung von Mitteln zur Bekämpfung von pflanzenpathogenen Schadpilzen, **dadurch gekennzeichnet, dass** man substituierte Benzoxa(thia)zole der Formel (I) gemäß Anspruch 1 oder 2 mit Streckmitteln und/oder oberflächenaktiven Stoffen vermischt.

7. Verfahren zum Herstellen der Verbindungen der Formel (I) umfassend wenigstens einen der folgenden Schritte der Verfahren A bis H: 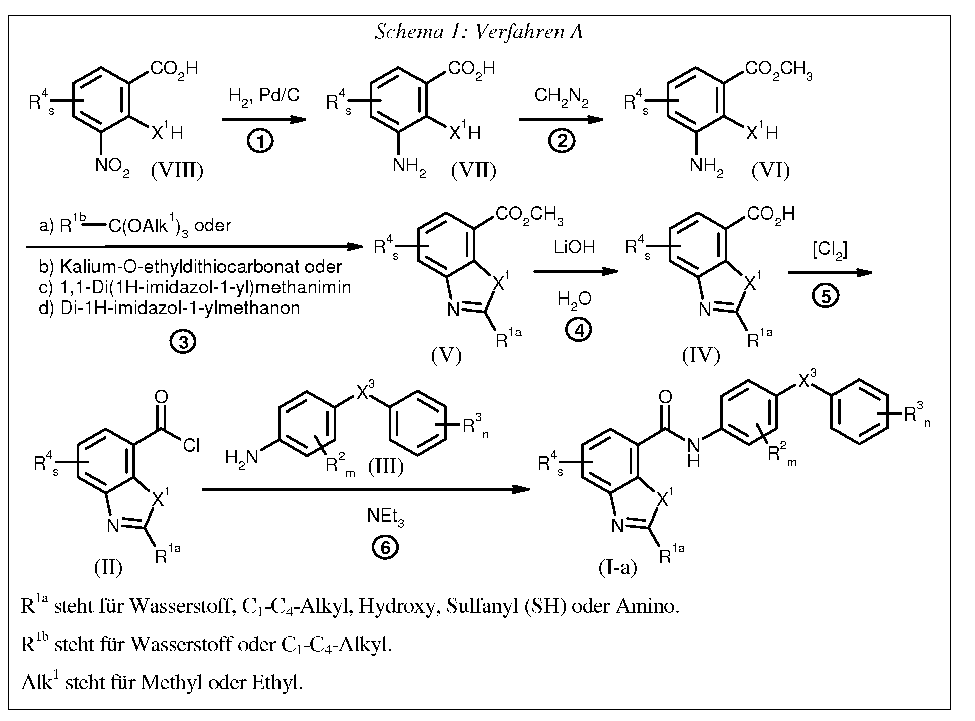 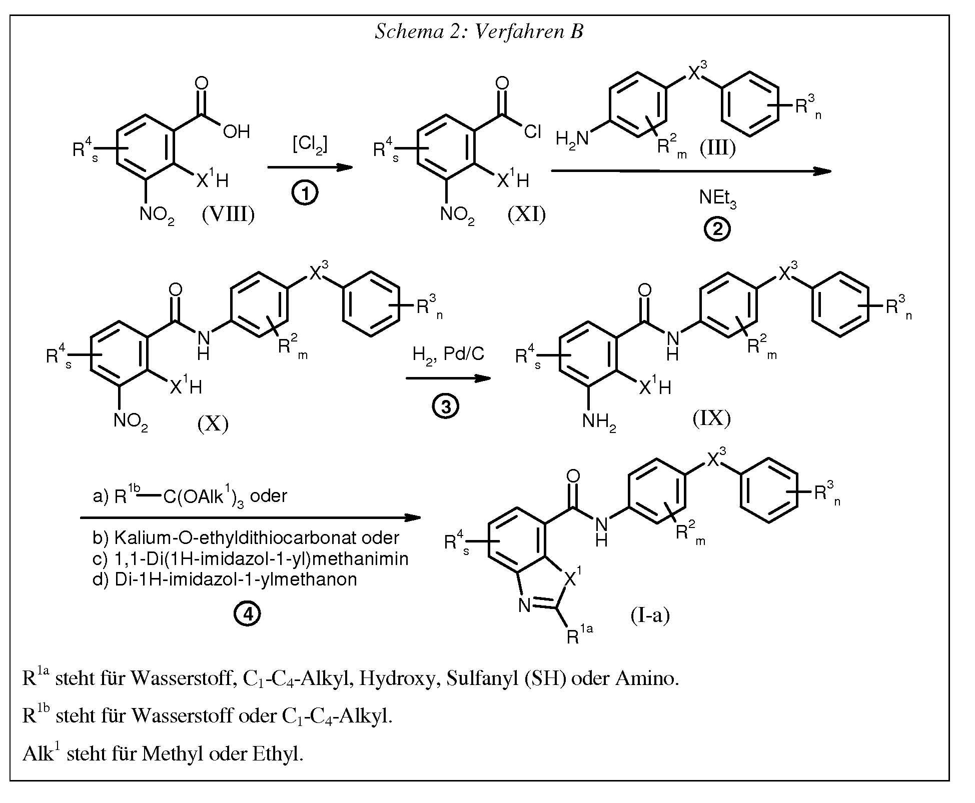 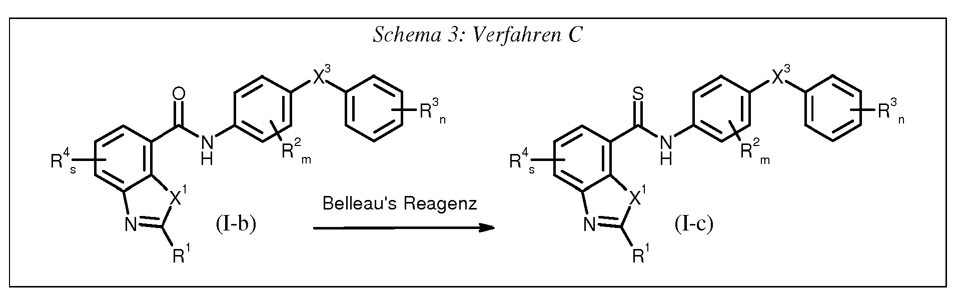 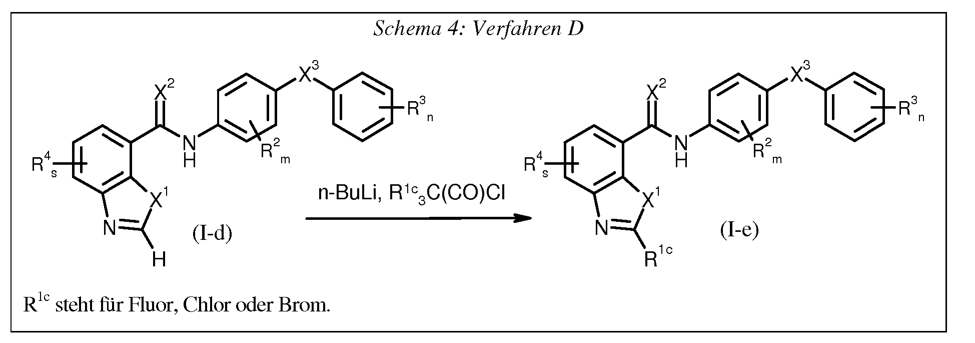 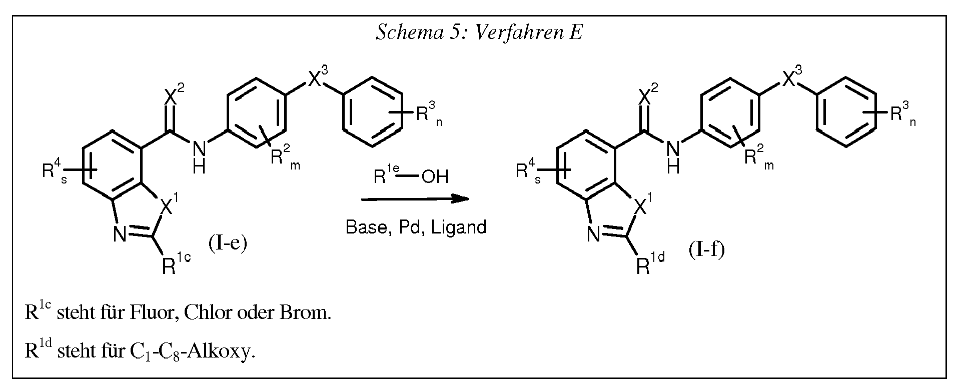 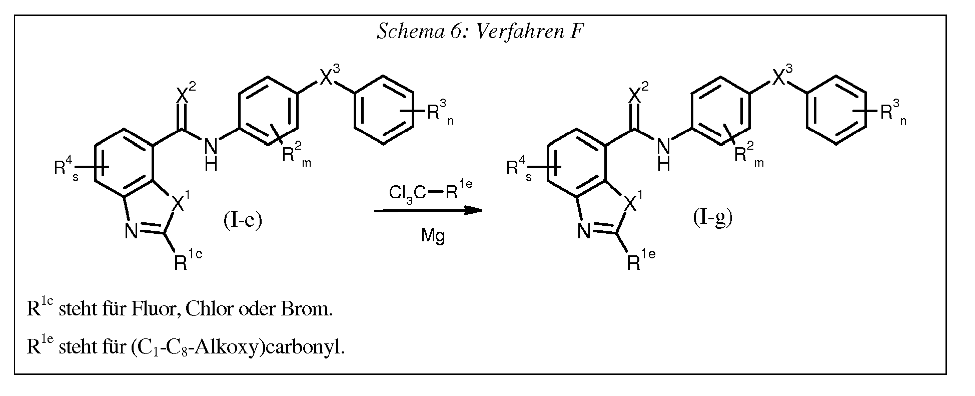 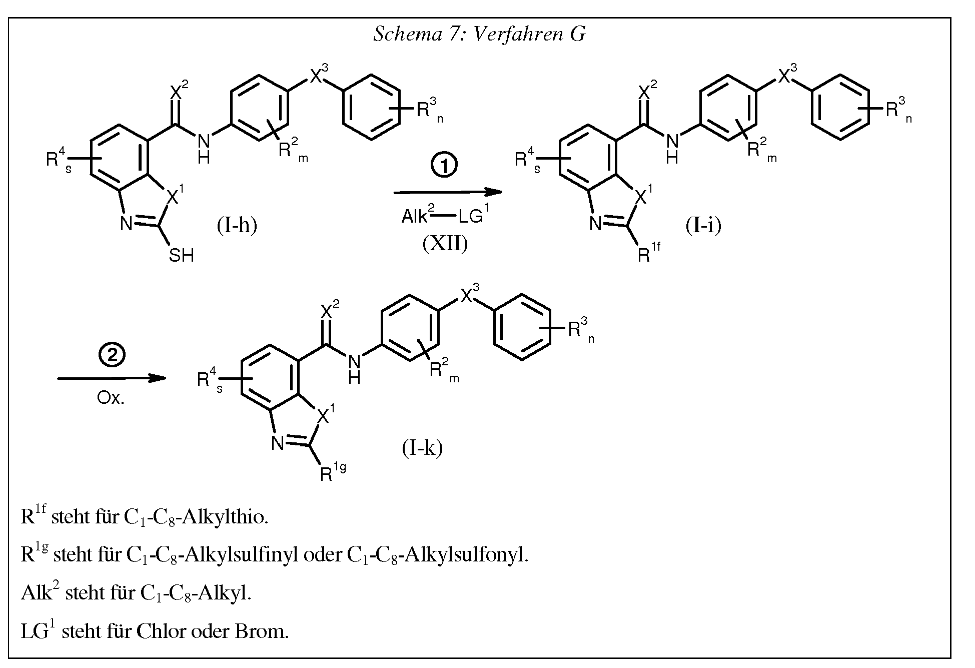 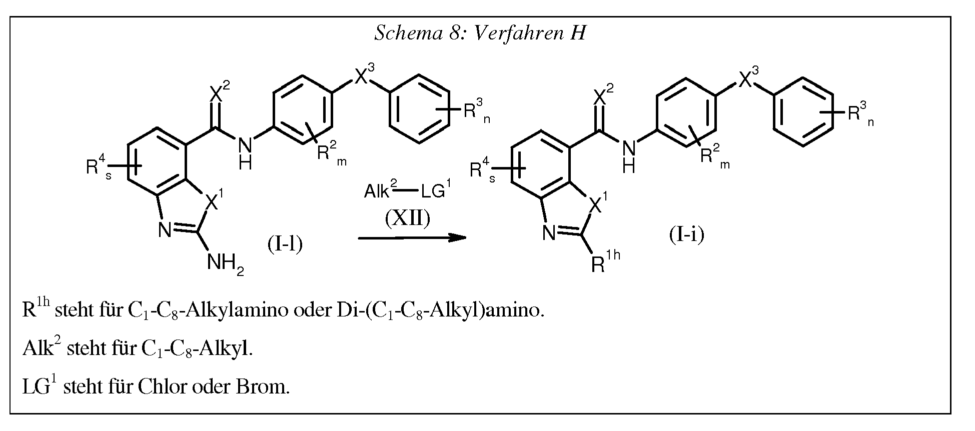 worin X¹, X², X³, R¹, R², m, R³, n, R⁴ und s die in Anspruch 1 angegebenen Bedeutungen haben.

8. Nitrobenzoesäure-Derivate der Formel (VIII-a), 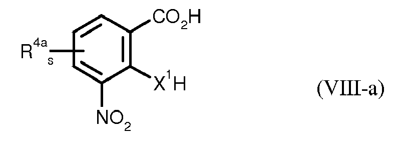
X¹ und s die oben angegebenen Bedeutungen haben,
R^{4a} für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C-₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.

9. Aminobenzoesäure-Derivate der Formel (VII-a), 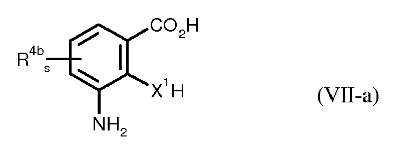 in welcher
X¹ und s die oben angegebenen Bedeutungen haben,
R^{4b} für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.

10. Verbindungen der Formel (VI-a), 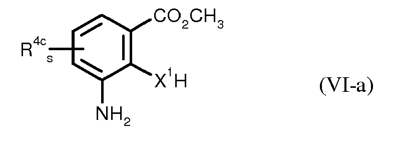 in welcher
X¹ und s die oben angegebenen Bedeutungen haben,
R^{4c} für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.

11. Benzoxa(thia)zolcarbonsäureester der Formel (V-a) 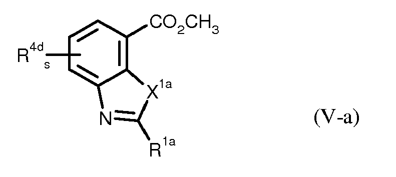 in welcher
X¹, R^{1a} und s die oben angegebenen Bedeutungen haben,
R^{4d} für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylanüno, Di-(C₁-C₈-alkyl)anüno, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinylC₁-C-₄-alkyl, Halogen-C₁-C₄-alkylsulfonyl-C₁-C₄-alkyl, Aryl, Hetaryl, Heterocyclyl steht,
sowie Salze davon.

12. Verbindungen der Formel (IV-a), 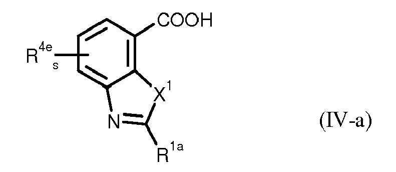 in welcher
X¹, R^{1a} und s die oben angegebenen Bedeutungen haben,
R^{4e} für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinylC₁-C₄-alkyl, Halogen-C₁-C₄-alkylsulfonyl-C₁-C₄-alkyl, Aryl, Hetaryl, Heterocyclyl steht,
sowie Salze davon.

13. Verbindungen der Formel (X-a), 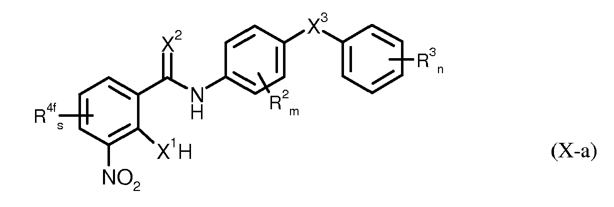 in welcher
X¹, X², X³, R², m, R³, n und s die oben angegebenen Bedeutungen haben,
R^{4f} für Halogen, Hydroxy, Cyano, Nitro, C₁-C₈-Alkyl, C₁-C₈-Alkoxy, C₁-C₈-Alkylthio, C₁-C₈-Alkylsulfinyl, C₁-C₈-Alkylsulfonyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkoxy, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Phenyl (welches wiederum gegebenenfalls einfach oder mehrfach, gleich oder verschieden durch Halogen, Cyano, Nitro, C₁-C₆-Alkyl, C₁-C₆-Alkoxy, C₁-C₆-Alkylthio, C₁-C₆-Halogenalkyl, C₁-C₆-Halogenalkylthio substituiert sein kann), Heteroaryl, Heterocyclyl steht.

14. Verbindungen der Formel (XI-a), 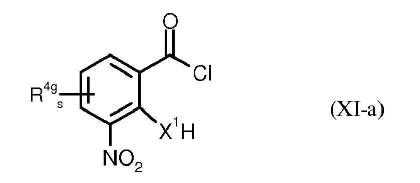 in welcher
X¹ und s die oben angegebenen Bedeutungen haben,
R^{4g} für C₁-C₈-Alkylsulfinyl, C₁-C₄-Alkoxy-C₁-C₄-alkyl, (C₁-C₄-Alkyl)thio(C₁-C₄-alkyl), (C₁-C₄-Alkyl)sulfinyl(C₁-C₄-alkyl), (C₁-C₄-Alkyl)sulfonyl(C₁-C-₄-alkyl), Formyl, C₁-C₈-Alkylcarbonyl, C₁-C₈-Alkylamino, Di-(C₁-C₈-alkyl)amino, C₁-C₈-Halogenalkyl, C₁-C₈-Halogenalkylthio, C₁-C₈-Halogenalkylsulfinyl, C₁-C₈-Halogenalkylsulfonyl, Halogen-C₁-C₄-alkoxy-C₁-C₄-alkyl, Halogen-C₁-C₄-alkylthio-C₁-C₄-alkyl, Halogen-C₁-C₄-alkylsulfinyl-C₁-C₄-alkyl, Halogen-C₁-C₄-alkylsulfonyl-C₁-C₄-alkyl, Aryl, Hetaryl, Heterocyclyl steht,
sowie Salze davon.

15. Verwendung von Verbindungen der Formel (I) gemäß Anspruch 1 oder 2 zur Behandlung von transgenen Pflanzen.
